# EUROPÄISCHE PATENTSCHRIFT

(11) **EP 1 742 599 B1**
(45) Veröffentlichungstag und Bekanntmachung des Hinweises auf die Patenterteilung: **15.06.2011**
(21) Anmeldenummer: 05749613.5
(22) Anmeldetag: 26.04.2005
(51) Int. Cl.: A61F 2/64

(54) **KNIEGELENKPROTHESE**
KNEE JOINT PROSTHESIS
PROTHESE D'ARTICULATION DE GENOU

(30) Priorität: 30.04.2004 DE 102004021250
(43) Veröffentlichungstag der Anmeldung: 17.01.2007
(73) Patentinhaber: TEKO Automation, Mensch und Technik GmbH, 78054 VS-Schwenningen (DE)
(72) Erfinder: BISINGER, Hardy, 72348 Rosenfeld (DE); MAREK, Volker, 78056 VS-Schwenningen (DE); FITZLAFF, Gerhard, 78054 VS-Schwenningen (DE); HOLTKAMP, Bernhard, 78166 Donaueschingen (DE)
(74) Vertreter: Hofer, Dorothea
(86) Internationale Anmeldenummer: PCT/DE2005/000764
(87) Internationale Veröffentlichungsnummer: WO 2005/104999

(56) Entgegenhaltungen:
- EP-A- 0 010 177
- GB-A- 1 091 015
- GB-A- 2 244 006
- US-A- 2 533 008
- US-A- 4 064 569
- US-A- 4 152 787

## Beschreibung

Die Erfindung betrifft eine Kniegelenkprothese nach dem Oberbegriff des Patentanspruchs 1.

Eine Kniegelenkprothese für eine Beinprothese ist zum Beispiel aus der OE 3241695 C2 bekannt. Die Kniegelenkprothese weist ein Gelenkoberteil und ein Gelenkunterteil auf, die über eine Gelenkachse miteinander verbunden sind. Zum Erzeugen einer Bremswirkung, die bei Belastung der Beinprothese mit dem Körpergewicht des Prothesenträgers ein Abknicken in dem Kniegelenk verhindert, sind zwei Bremsflächen vorgesehen, die mit einem Bremsbelag versehen sind und durch Verkippen gegeneinander bei Gewichtsbelastung eine Bremswirkung erzeugen. Eine solche Kniegelenkprothese ermöglicht jedoch keine genaue Ansteuerung der Bremseinrichtung durch die Größe der einwirkenden Körperkraft. Das Auslösen der Bremse ist zudem unabhängig davon, in welcher Phase eines Schrittes sich die Beinprothese befindet, d.h. die Stärke der Bremswirkung tritt unabhängig davon auf, ob z.B. die Spitze des Fußes der Beinprothese belastet wird oder die Ferse. Eine Unterstützung eines natürlichen Gangs kann somit nicht erfolgen.

Ein weiterer Stand der Technik ist in US-A-4 152 787 angegeben.

Es sind weiterhin Kniegelenkprothesen mit einer so genannten Wickelbremse bekannt, bei der bei Körperbelastung der Radius eines koaxial zu der Gelenkachse angeordneten geschlitzten Zylinders verjüngt wird, sodass die Innenseite dieses Zylinders auf die Gelenkachse einwirkt und so eine Bremswirkung erzeugt wird. Bei diesen Wickelbremsen tritt jedoch das Problem auf, dass die Bremse beim Entlasten die Beugebewegung nicht unmittelbar wieder freigibt, also ein selbsthemmender Effekt auftritt. Dieser Effekt führt dazu, dass sich die Prothesenträger eine unnatürliche Gangart aneignen, bei der beim Bewegen der Hüfte nach vorne im Laufe eines Schrittes diese immer zusätzlich angehoben wird, um die Wickelbremse wieder frei zu geben.

Aufgabe der Erfindung ist es, eine verbesserte Kniegelenkprothese zu schaffen.

Die Aufgabe wird gelöst durch eine Kniegelenkprothese nach Anspruch 1. Weiterbildungen der Erfindung sind in den Unteransprüchen gekennzeichnet.

Die Kniegelenkprothese hat insbesondere den Vorteil, dass das Blockieren der Bremseinrichtung bei Entlastung sofort gelöst und zugleich eine belastungsabhängige zur eingeleiteten Gewichtskraft proportionale Bremskraft erzielt wird.

Weitere Merkmale und Zweckmäßigkeiten der Erfindung ergeben
sich aus der Beschreibung von Ausführungsbeispielen anhand der beigefügten Zeichnungen. Von den Figuren zeigen:
Fig. 1a bis 1c eine schematische Darstellung einer Beinprothese mit einem Kniegelenk einer Ausführungsform der vorliegenden Erfindung in drei verschiedenen Beinstellungen;
Fig. 2 eine Vorderansicht eines Kniegelenks einer Ausführungsform der vorliegenden Erfindung in gestreckter Stellung;
Fig. 3 eine Seitenansicht des in Fig. 2 dargestellten Kniegelenks;
Fig. 4 eine Rückansicht des in Fig. 2 dargestellten Kniegelenks;
Fig. 5 eine teilgeschnittene Seitenansicht des in Fig. 2 dargestellten Kniegelenks;
Fig. 6 einen Schnitt entlang der Linie B-B in Fig. 3 durch einen oberen Teil des Kniegelenks;
Fig. 7 einen Schnitt entlang der Linie c-c in Fig. 3 durch einen oberen Teil des Kniegelenks;
Fig. 8 einen Schnitt entlang der Linie A-A in Fig. 3 durch einen oberen Teil des Kniegelenks;
Fig. 9a eine Vorderansicht des ersten und des zweiten Kraftübertragungselements des Kniegelenks gemäß einer Ausführungsform der vorliegenden Erfindung;
Fig. 9b eine perspektivische Darstellung des ersten und des zweiten Kraftübertragungselements;
Fig. 9c eine perspektivische Darstellung des ersten und des zweiten Kraftübertragungselements im zusammengesetzten Zustand;

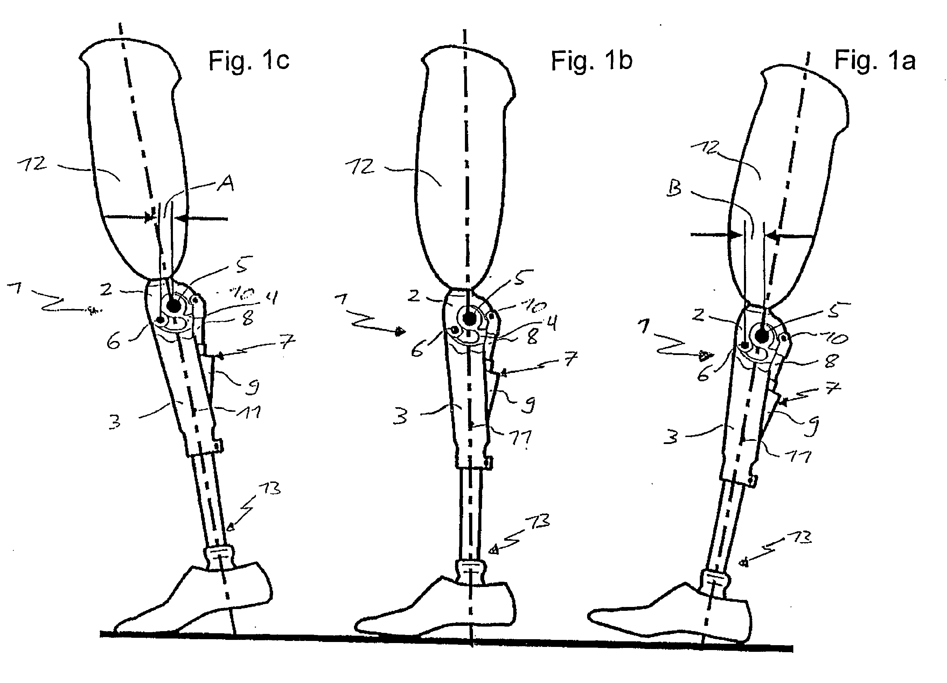

In den Fig. 1a bis 1c ist ein Kniegelenk, im Weiteren auch als Bremskniegelenk bezeichnet, einer Ausführungsform der vorliegenden Erfindung in einer Beinprothese dargestellt. Wie in Fig. I zu erkennen ist, weist das Bremskniegelenk I ein Gelenkoberteil 2, ein Gelenkunterteil 3 und ein dazwischen angeordnetes Gelenkmittelteil 4 auf. Das Gelenkmittelteil 4 ist um eine Gelenkachse 5 drehbar mit dem Gelenkunterteil 3 verbunden. Das Gelenkoberteil 2 ist mit dem Gelenkmittelteil 4 um eine Schwingachse 6 schwenkbar verbunden. Die Schwingachse 6 ist parallel zu der Gelenkachse 5 ausgerichtet und streckseitig und nach unten versetzt bezüglich dieser angeordnet.

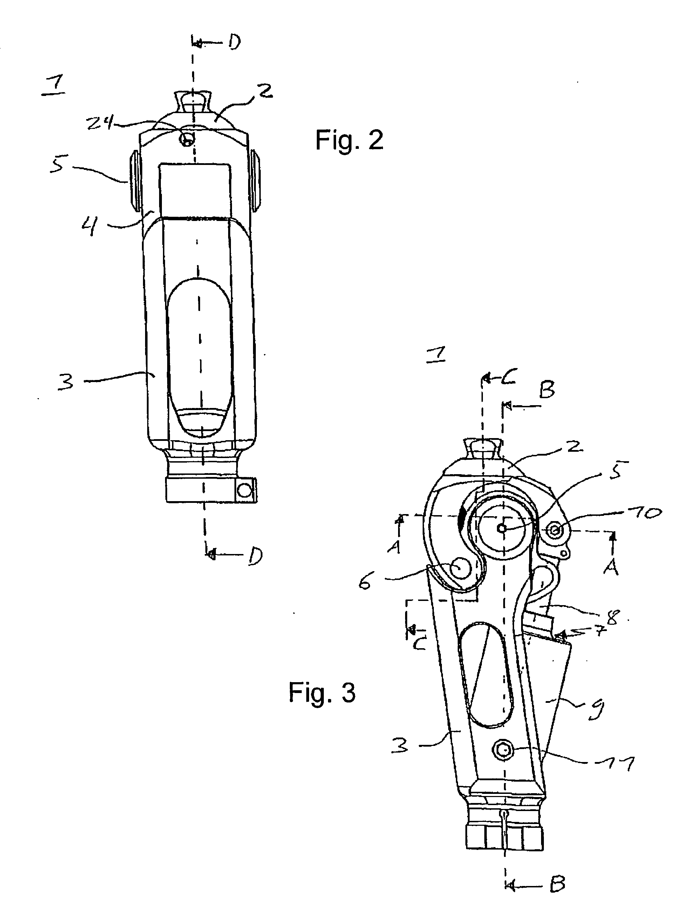

Wie insbesondere aus den Fig. 2 bis 5 zu ersehen ist, ist das Gelenkoberteil 2 weiterhin über ein Schwungphasensteuerungselement 7 an das Gelenkunterteil 3 angelenkt. Das Schwungphasensteuerungselement 7 ist als ein Kolben-Zylinder-System ausgebildet, bei dem eine Kolbenstange 8 in einen Zylinder 9 hineingeschoben und wieder aus diesem herausgezogen werden kann. Die Anlenkung erfolgt über eine drehbare Befestigung der Kolbenstange 8 des Schwungphasensteuerungselements 7 an dem Gelenkoberteil 2 mit einer Drehachse 10 und eine drehbare Befestigung des Zylinders 9 des Schwungphasensteuerungselements 7 an dem Gelenkunterteil 3 mit einer Drehachse 11.

Das Schwungphasensteuerungselement 7 ist vorzugsweise als eine pneumatische Kolben-Zylinder-Einrichtung ausgebildet, die die Bewegung der Kolbenstange 8 relativ zu dem Zylinder 9 abhängig von der Bewegungsgeschwindigkeit dämpft. Alternativ ist auch die Verwendung einer hydraulischen Kolben-Zylinder-Einrichtung oder einer ähnlichen Vorrichtung denkbar, die die Bewegung der Kolbenstange 8 relativ zu dem Zylinder 9 dämpft.

In dem in den Fig. 1a bis 1c dargestellten montierten Zustand des Bremskniegelenks 1 in einer Beinprothese schließt an das Gelenkoberteil 2 ein Oberschenkelteil 12 und an das Gelenkunterteil 3 ein Fußteil 13 an. Das Gelenk ist jeweils in einem gestreckten Zustand dargestellt.

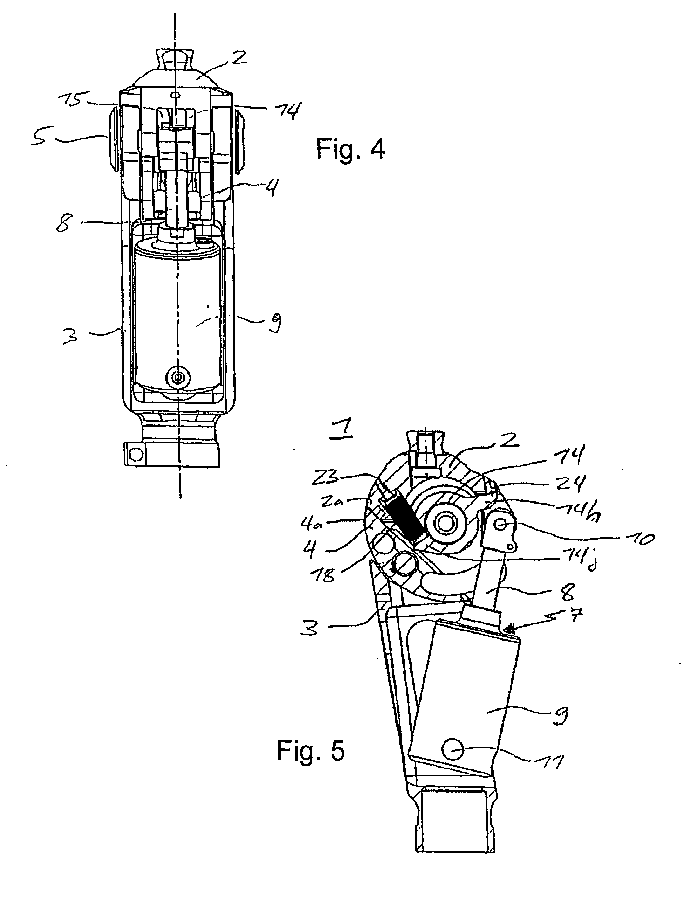

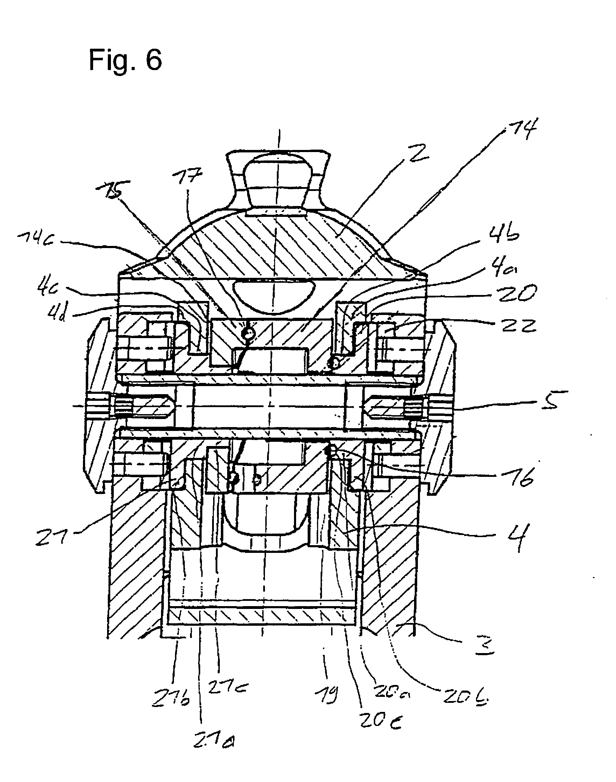

Anhand der Figuren 5 bis 9b wird im Folgenden der Aufbau der Bremseinrichtung der Kniegelenkprothese beschrieben. Wie aus den Fig. 5 und 6 zu erkennen ist, sind im Wesentlichen koaxial zu der Gelenkachse 5 im Inneren des Bremskniegelenks ein erstes Kraftübertragungselement 14 und ein zweites Kraftübertragungselement 15 angeordnet.

Das erste Kraftübertragungselement 14 ist um die Gelenkachse 5 bezüglich des Gelenkmittelteils 4 drehbar angeordnet. Das zweite Kraftübertragungselement 15 ist begrenzt entlang der Gelenkachse 5 bewegbar, es ist jedoch drehfest bezüglich der Gelenkachse 5 mit dem Gelenkmittelteil 4 verbunden.

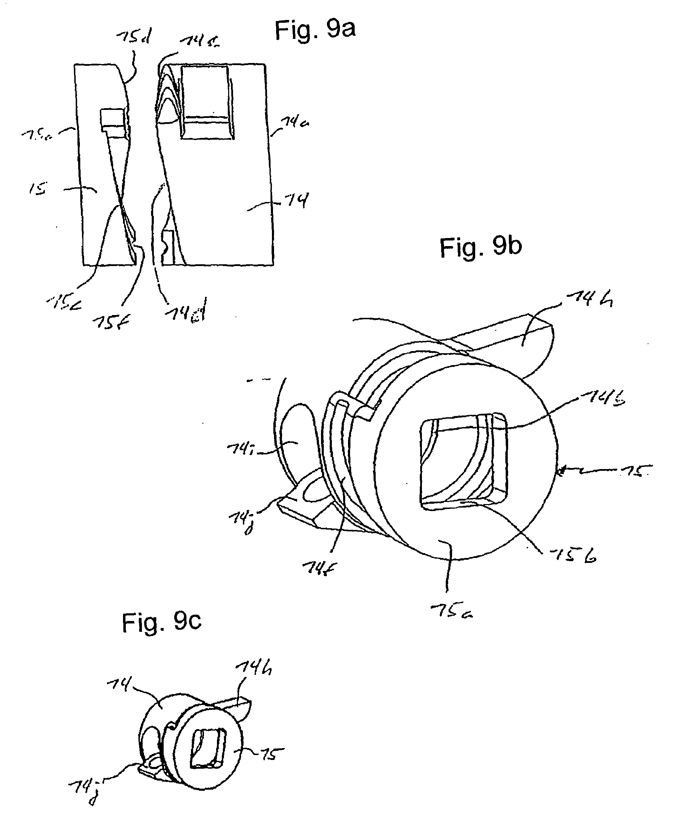

Anhand der Fig. 9a und 9b werden nun in Verbindung mit Fig. 6 das erste Kraftübertragungselement 14 und das zweite Kraftübertragungselement 15 detailliert beschrieben.

Wie in den Fig. 6 und 9a bis 9c gezeigt ist, ist das erste Kraftübertragungselement 14 im Wesentlichen zylindrisch mit einer kreisförmigen koaxialen Bohrung 14b ausgebildet. Die erste Stirnfläche 14a ist kreisförmig mit einer im Wesentlichen ebenen Oberfläche ausgebildet. In der Stirnfläche 14a ist eine koaxial ringsumlaufende Rille 14e ausgebildet, die zur Führung erster Kugellagerkugeln 16 dient. Die zweite Stirnfläche 14c weist in der dargestellten Ausführungsform drei spindelförmig ansteigende um die koaxiale Bohrung 14b umlaufende Flächen 14d auf. Der spindelförmige Anstieg ist dabei derart, dass beim kreisförmigen Umlaufen entgegen dem Uhrzeigersinn um die Bohrung 14b auf der zweiten Stirnfläche 14c der Abstand zu der ersten Stirnfläche immer größer wird bis das Ende der jeweiligen Fläche 14d erreicht ist und der Abstand dann in Form einer Stufe zum Anfang der nächsten Fläche wieder auf den Ausgangswert abnimmt. Auf der zweiten Stirnfläche 14c ist koaxial umlaufend in den Flächen 14d eine Rille 14f zur Führung zweiter Kugellagerkugeln 17 ausgebildet.

Von dem Zylindermantel 14g ausgehend befindet sich auf einer Seite eine erste Nocke 14h, die im Wesentlichen von dem Zylindermantel radial nach außen ragt und der Krafteinbringung dient, die später noch ausführlich beschrieben wird. Auf der anderen Seite des Zylindermantels 14g befindet sich eine in im Wesentlichen tangentialer Richtung ausgebildete Ausnehmung 14i, die eine zweite Nocke 14j bildet, die etwas aus dem Zylindermantel ragt und deren Nockenfläche zum Angreifen eines Federelements zum Einstellen der Auslösekraft dient.

Das zweite Kraftübertragungselement 15 ist ebenfalls im Wesentlichen zylindrisch mit einer koaxialen Bohrung mit im Wesentlichen quadratischem Querschnitt ausgebildet. Eine erste Stirnfläche 15a des zweiten Kraftübertragungselements 15 ist ebenso wie die Zylindermantelfläche mit einer ebenen Oberfläche ausgebildet. Die zweite Stirnfläche 15c weist analog zu der zweiten Stirnfläche 14c des ersten Kraftübertragungselements 14 spindelförmig ansteigende Flächen 15d und eine Rille 15f auf.

Fig. 9c zeigt eine perspektivische Darstellung des ersten und des zweiten Kraftübertragungselements 14, 15 im zusammengesetzten Zustand. Zwischen den Rillen 14f, 15f befinden sich in diesem Zustand die zweiten Kugellagerkugeln 17, die in Fig. 9c nicht dargestellt sind, jedoch in Fig. 6 gezeigt sind. Wie in den Fig. 9a bis 9c zu erkennen ist, sind die ersten Stirnflächen 14a, 15a parallel zueinander ausgerichtet. Es ist in Fig. 9c zu erkennen, dass der Abstand zwischen den ersten Stirnflächen 14a, 15a zunimmt, wenn das zweite Kraftübertragungselement 15 ortsfest gehalten wird und das erste Kraftübertragungselement 14 um die Zylinderachse im Uhrzeigersinn gedreht wird. Somit bilden das erste und das zweite Kraftübertragungselement 14, 15 eine Hubspindel.

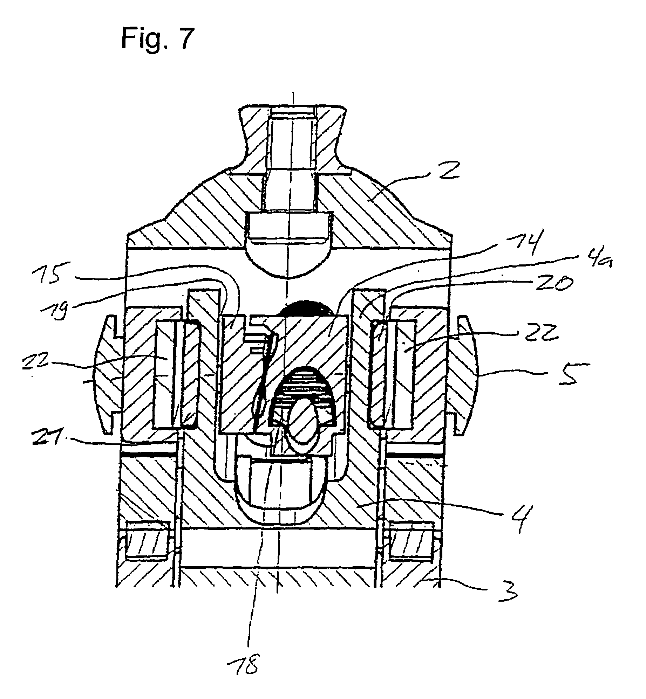

Wie aus Fig. 7 ersichtlich ist, ist das Gelenkmittelteil 4 derart ausgebildet, dass es zwei freie Schenkel 4a bildet, zwischen denen das erste 14 und das zweite Kraftübertragungselement 15 im zusammengebauten Zustand eingesetzt sind. Zwischen den ersten Stirnflächen 14a, 15a und den freien Schenkeln 4a, 4a sind dabei Luftspalte 19 freigelassen.

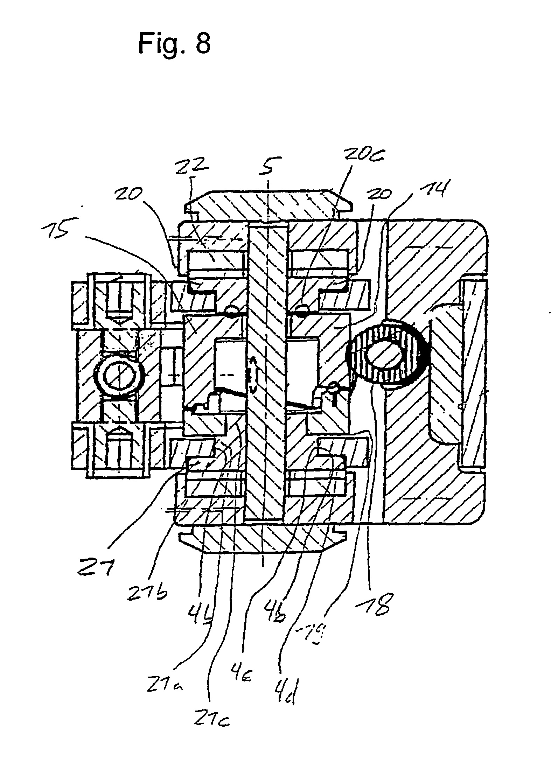

Wie insbesondere aus den Fig. 6 und 8 zu ersehen ist, sind in den freien Schenkeln 4a, 4a Bohrungen 4b, 4b vorgesehen, die koaxial zu der Gelenkachse 5 angeordnet sind und in einem ersten Abschnitt 4c mit kleinerem Querschnitt je einen im Wesentlichen quadratischen Querschnitt aufweisen. In einem zweiten Abschnitt 4d weisen diese Bohrungen je einen größeren kreisförmigen Querschnitt auf. Durch diese Bohrungen 4b, 4b hindurchgreifend sind koaxial zu der Gelenkachse 5 angeordnete Bremsscheiben 20, 21 vorgesehen.

Die dem ersten Kraftübertragungselement 14 zugewandte Bremsscheibe 20 weist einen ersten Abschnitt 20a mit einem kleineren, im Wesentlichen quadratischen Querschnitt senkrecht zur Gelenkachse 5 und einen zweiten Abschnitt 20b mit einem größeren, kreisförmigen Querschnitt auf. Der erste Abschnitt 20a ist derart ausgebildet, dass eine Bewegung in der axialen Richtung der Gelenkachse 5 in der Bohrung 4b möglich ist, jedoch keine Rotation der Bremsscheibe 20 um die Gelenkachse 5 bezüglich des Gelenkmittelteils 4. Der Radius des zweiten Abschnitts 20b ist etwas kleiner als der Radius des zweiten Abschnitts 4d der Bohrung 4b, sodass er teilweise in diesen versenkbar ist. Auf der dem ersten Kraftübertragungselement 14 zugewandten Seite weist die Bremsscheibe 20 weiterhin eine zu der Gelenkachse 5 koaxial verlaufende Rille 20c zum Führen der ersten Kugellagerkugeln 16 auf.

Die dem zweiten Kraftübertragungselement 15 zugewandte Bremsscheibe 21 weist, wie aus den Fig. 6 und 8 zu erkennen ist, ebenfalls einen ersten Abschnitt 21a und einen zweiten Abschnitt 21b auf, die wie der erste 20a und der zweite Abschnitt 20b der Bremsscheibe 20 ausgebildet sind. Die Bremsscheibe 21 weist jedoch anschließend an den zweiten Abschnitt 21b auf der dem zweiten Kraftübertragungselement 15 zugewandten Seite weiterhin einen dritten Abschnitt 21c auf, der einen im Wesentlichen quadratischen, kleineren Querschnitt als der zweite Abschnitt 21b aufweist. Der Querschnitt des dritten Abschnitts 21c ist gerade so gewählt, dass ein Eingreifen in die im Wesentlichen quadratische Bohrung 15b des zweiten Kraftübertragungselements 15 erfolgen kann und dieses gegen Verdrehen gegenüber der Bremsscheibe 21 gesichert ist. Der Querschnitt des zweiten Abschnitts 21b ist analog zu dem zweiten Abschnitt 20b der Bremsscheibe 20 so gewählt, dass ein Eingreifen in die im Wesentlichen quadratische Bohrung 4b des Gelenkmittelteils 4 derart erfolgen kann, dass eine Bewegung in der axialen Richtung der Gelenkachse 5 in der Bohrung 4b möglich ist, jedoch keine Rotation der Bremsscheibe 20 um die Gelenkachse 5 bezüglich des Gelenkmittelteils 4.

Angrenzend an die den Kraftübertragungselementen 14, 15 abgewandten Seitenflächen der Bremsscheiben 20, 21 befinden sich in dem Gelenkunterteil befestigte bevorzugt als Stahlscheiben ausgebildete Scheiben 22 mit Bremsbelag.

Wie in Fig. 5 zu erkennen ist, drückt die zweite Nocke 14j des ersten Kraftübertragungselements 14 gegen das Federelement 18, dessen Gegenlager von einer Federkraft-Einstellschraube 23 gebildet wird, die mit dem Gelenkoberteil 2 über ein Gewinde verbunden ist. Die Gegenkraft zu der durch das Federelement 18 auf das um die Gelenkachse 5 drehbar gelagerte erste Kraftübertragungselement 14 ausgeübten Kraft bildet die auf die erste Nocke 14h einwirkende Spielausgleichsschraube 24, die über ein Gewinde mit dem Gelenkoberteil 2 verbunden ist.

Der zuvor beschriebene Aufbau ermöglicht es, dass das erste Kraftübertragungselement 14 über die ersten 16 und zweiten Kugellagerkugeln 17 gelagert gegenüber dem Gelenkmittelteil 4 und dem in diesem verdrehgesichert gehaltenen zweiten Kraftübertragungselement 15 verdreht werden kann. Durch das Hubspindelprinzip des Zusammenwirkens des ersten 14 und des zweiten Kraftübertragungselements 15 werden dabei die ersten Stirnflächen 14a, 15a und über diese die beiden Bremsscheiben 20, 21 nach außen bewegt. Das erste 14 und das zweite Kraftübertragungselement 15 sowie die Bremsscheiben 20, 21 werden dabei von der in dem Gelenkunterteil 3 gehaltenen Gelenkachse 5 in dem Gelenkmittelteil 4 geführt. Die nach außen bewegten Bremsscheiben 20, 21 pressen gegen die in dem Gelenkunterteil 3 befestigten Scheiben 22 und bremsen dadurch eine Bewegung des Gelenkmittelteils 4 gegenüber dem Gelenkunterteil 3 um die Gelenkachse 5.

Anhand von Fig. 3 wird nun zuerst ein Beugen des Bremskniegelenks 1 ohne ein Auslösen der Bremseinrichtung beschrieben.

Beim langsamen Beugen des Bremskniegelenks mit geringer Krafteinwirkung wird das Gelenkmittelteil 4 um die Gelenkachse 5 im Uhrzeigersinn gegenüber dem Gelenkunterteil 3 verdreht. Bei dieser Drehbewegung bewegt sich die Schwingachse 6 ebenfalls im Uhrzeigersinn um die Gelenkachse 5. Die Schwingachse bewegt sich in Fig. 3 also auf einer Kreisbahn nach links oben. Bei geringer Krafteinwirkung bewegt sich das an der Schwingachse 6 an das Gelenkmittelteil 4 angekoppelte Gelenkoberteil 2 ebenfalls im Uhrzeigersinn mit dem Gelenkmittelteil 4 mit, sodass sich die Drehachse 10 ebenfalls auf einer kreisförmigen Bahn im Uhrzeigersinn um die Gelenkachse 5 bewegt. Die Drehachse 10 bewegt sich in Fig. 3 also im Wesentlichen nach unten.

Bei Bewegung der Drehachse 10 nach unten wird die Kolbenstange 8 in den Zylinder 9 hineingeschoben und es findet eine leichte Drehbewegung des Schwungphasensteuerungselements 7 um die Drehachsen 10 und 11 relativ zu dem Gelenkoberteil 2 bzw. dem Gelenkunterteil 13 statt.

Bei einer Streckung des Bremskniegelenks findet die zuvor beschriebene Bewegung in umgekehrter Richtung statt.

Im Folgenden wird Bezug nehmend auf Fig. 5 die Ansteuerung der Bremseinrichtung durch Fußbelastung beschrieben.

Mit der Federkraft-EinsteIlschraube 23 ist über das Federelement 18 eine vorbestimmte Auslösekraft eingestellt. Diese wird abhängig von dem Gewicht und der Aktivität des Prothesenträgers eingestellt.

Wenn keine vertikale Kraft von oben auf das Gelenkoberteil 2 wirkt und das Federelement 18 eine Vorspannung aufweist, dann wird das Gelenkoberteil 2, das um die Schwingachse 6 zu dem Gelenkmittelteil 4 drehbar ist, durch die erste Nocke 14h mit der Kante 2a gegen den Anschlag 4a des Gelenkmittelteils 4 gedrückt. Das erste 14 und das zweite Kraftübertragungselement 15 befinden sich in dieser Situation in einem Ruhezustand, in dem die Bremsscheiben 20, 21 nicht nach außen gedrückt werden und folglich die Rotation des Gelenkmittelteils 4 relativ zu dem Gelenkunterteil 3 um die Gelenkachse 5 nicht gebremst wird.

Wirkt eine vertikale Kraftkomponente von oben derart auf das Gelenkoberteil 2 ein, dass das von der Spielausgleichsschraube 24 über die erste Nocke 14h auf das erste Kraftübertragungselement 14 übertragene Drehmoment das Drehmoment überwiegt, das von dem Federelement 18 über die zweite Nocke 14j übertragen wird, so verkippt das Gelenkoberteil 2 gegenüber dem Gelenkmittelteil 4 um die Schwingachse 6. Bei dem Verkippen wird das erste Kraftübertragungselement 14 gegenüber dem zweiten Kraftübertragungselement 15 um die Gelenkachse 5 verdreht und folglich werden wie bereits oben beschrieben die Bremsscheiben 20, 21 nach außen bewegt und eine Drehung des Gelenkmittelteils 4 um die Gelenkachse 5 relativ zu dem Gelenkunterteil 3 gebremst.

Wird die vertikale Kraftkomponente wieder aufgehoben, so drückt die Tellerfeder 18 das erste Kraftübertragungselement 14 wieder zurück und das Gelenkoberteil 2 somit wieder in den Anschlag 4a am Gelenkmittelteil 4, sodass die Bremse wieder freigegeben wird.

Die Auslösekraft der Scheibenbremse kann über die Federkraft- Einstellschraube 23 justiert werden, indem die Vorspannung der Tellerfeder 18 durch Verdrehen der Federkraft-Einstellschraube 23 entweder erhöht oder verringert wird.

Über die Spielausgleichsschraube 24 kann ein Vorverdrehen des ersten Kraftübertragungselements 14 gegenüber dem zweiten Kraftübertragungselement 15 erreicht werden, sodass bei einem auftretenden Verschleiß der Bremsbeläge eine einfache Nachjustierung erfolgen kann.

Wie anhand der Fig. 1a bis 1c zu sehen ist, weist die zuvor beschriebene Kniegelenkprothese aufgrund der besonderen Anordnung der Schwingachse 6 in einer Anordnung parallel, streckseitig und auf der Seite des Gelenkunterteils bezüglich der Gelenkachse 5 nach unten versetzt den besonderen Vorteil auf, dass die Bremswirkung der Scheibenbremse abhängig davon ist, in welcher Phase eines Schrittes sich die Beinprothese befindet.

In der Fig. 1a ist zu erkennen, dass die Schwingachse 6 beim Aufsetzen der Ferse der Beinprothese bezüglich einer vertikal von oben angreifenden Kraft einen großen Hebel B zu der Gelenkachse 5 bildet, d.h. die Bremswirkung bei einer relativ geringen einwirkenden Körperbelastung der Beinprothese einsetzt. Wie in der Fig. 1c gezeigt ist, ist der Hebel A, der beim Aufsetzen der Fußspitze gebildet ist, wesentlich kleiner, sodass für die Bremswirkung eine größere Körperbelastung der Beinprothese erforderlich ist, d.h. die Bremse beim Entlasten früher freigibt. Dieser Effekt ermöglicht besonders in Verbindung mit der Scheibenbremse eine natürlichere Bewegung des Beins beim Gehen, da Vermieden wird, dass die Bremse beim Einleiten der Schwungphase des Beines noch greift. Zugleich wird eine hohe Stabilität der Beinprothese in der Standphase erreicht.

Das Verwenden der Scheibenbremse führt zu einer deutlichen Reduktion der Abnutzung der Bremseinrichtung, sodass der Abstand erforderlicher Wartungsintervalle reduziert werden kann und die Zuverlässigkeit der Kniegelenkprothese deutlich verbessert ist.

Die Ausführung der Kniegelenkprothese mit den beiden Kraftübertragungselementen 14, 15 führt außerdem dazu, dass die Stärke der Bremswirkung durch die Dosierung der einwirkenden vertikalen Kraftkomponente durch den Prothesenträger gesteuert werden kann, sodass die Eigenschaften der Kniegelenkprothese deutlich verbessert sind. Bei der Verwendung in Kombination mit einem pneumatisch arbeitenden Schwungphasensteuerungselement 7 kann weiterhin das Gewicht der Kniegelenkprothese niedrig gehalten und folglich der Tragekomfort erhöht werden.

Es ist ebenfalls denkbar, z.B. das erste Kraftübertragungselement in Form eines Keils auszubilden, der bei Einwirken der auslösenden Kraft zwischen zwei schiefe Ebenen getrieben wird und diese dabei zum Ausüben der Kraft auf eine oder Bremsscheiben nach außen gedrückt werden. Bei einer solchen Vorrichtung wird der Keil dann bei Entlastung wieder ausgetrieben und folglich keine Bremskraft mehr auf die Bremsscheiben ausgeübt.

Ebenso ist es denkbar, die Kraftübertragung über ein Winkelhebelsystem zu realisieren. Auch ist es denkbar, die Kraftübertragung über eine hydraulische Vorrichtung zu realisieren, bei der die auslösende Kraft auf einen Kolben wirkt und diese auf einen zweiten Kolben übertragen wird, der dann die übertragene Kraft auf die Bremsscheibe ausübt.

## Patentansprüche

1. Bremskniegelenk für eine Beinprothese mit einem Gelenkoberteil (2), einem um eine Gelenkachse (5) relativ zu dem Gelenkoberteil (2) drehbaren Gelenkunterteil (3), und einer Bremseinrichtung (14, 15, 20, 21), die ein koaxial zur Gelenkachse (5) angeordnetes erstes Kraftübertragungselement (14) aufweist, auf das eine die Bremswirkung auslösende Kraft wirkt, und das die Kraft unter einem vorgegebenen Übertragungsverhältnis auf ein koaxial zur Gelenkachse (5) angeordnetes zweites Kraftübertragungselement (15) umsetzt, das die umgesetzte Kraft in bezüglich der Gelenkachse (5) im wesentlichen paralleler Richtung auf eine Bremsscheibe (20, 21) ausübt,
**dadurch gekennzeichnet, dass**
das erste Kraftübertragungselement (14) und das zweite Kraftübertragungselement (15) einander zugewandte Stirnflächen (14c, 15c) aufweisen, die jeweils spindelförmig ansteigende, umlaufende Flächen (14d, 15d) aufweisen.

2. Bremskniegelenk nach Anspruch 1, bei dem das erste und das zweite Kraftübertragungselement (14, 15) eine schiefe Ebene zur Umsetzung der Kraft ausbilden.

3. Bremskniegelenk nach einem der Ansprüche 1 bis 2, mit einem zwischen dem Gelenkoberteil (2) und dem Gelenkunterteil (3) vorgesehenen Gelenkmittelteil (4), wobei das Gelenkmittelteil (4) mit entweder dem Gelenkoberteil (2) oder dem Gelenkunterteil (3) über die Gelenkachse (5) verbunden ist und mit dem jeweils anderen über eine Schwingachse (6) verbunden ist.

4. Bremskniegelenk nach Anspruch 3, bei dem die Schwingachse (6) bezüglich der Gelenkachse (5) parallel, streckseitig und auf der Seite des Gelenkunterteils (3) angeordnet ist.

5. Bremskniegelenk nach einem der Ansprüche 1 bis 4, bei dem das erste und das zweite Kraftübertragungselement (14, 15) derart ausgebildet sind, dass das erste Kraftübertragungselement (14) durch die die Bremswirkung auslösende Kraft um eine zu der Gelenkachse (5) im Wesentlichen parallele Rotationsachse gedreht wird und die umgesetzte Kraft das zweite Kraftübertragungselement (15) in im Wesentlichen zu der Gelenkachse (5) parallele Translation versetzt.

6. Bremskniegelenk nach einem der Ansprüche 1 bis 5, bei dem das erste Kraftübertragungselement (14) und das zweite Kraftübertragungselement (15) eine auf der Gelenkachse (5) angeordnete Hubspindel bilden.

7. Bremskniegelenk nach einem der Ansprüche 1 bis 4, bei dem die Kraftübertragung über ein hydraulisches System erfolgt.

8. Bremskniegelenk nach einem der Ansprüche 1 bis 4, bei dem die Kraftübertragung über einen Winkelhebel erfolgt.

9. Bremskniegelenk nach einem der Ansprüche 1 bis 8, bei dem in Richtung der Gelenkachse (5) auf beiden Seiten des zweiten Kraftübertragungselements (15) Bremsscheiben (20, 21) vorgesehen sind.

10. Bremskniegelenk nach einem der Ansprüche 1 bis 9, bei dem die Voreinstellung der die Bremswirkung auslösenden Kraft durch eine vorgespannte Feder (18) erreicht wird.

11. Bremskniegelenk nach einem der Ansprüche 1 bis 10, bei dem zur Dämpfung bei der Gehbewegung eine Kolben-Zylinder-Einrichtung (7) vorgesehen ist.

12. Bremskniegelenk nach einem der Ansprüche 1 bis 11, bei dem die Bremseinrichtung (14, 15, 20, 21) durch Fußbelastung ansteuerbar ist.

13. Bremskniegelenk nach einem der Ansprüche 1 bis 12, bei dem die auf das erste Kraftübertragungselement (14) einwirkende Kraft im Wesentlichen senkrecht bezüglich der Gelenkachse einwirkt.

## Claims

1. A knee joint with brake for an artificial leg, comprising an upper joint part (2), a lower joint part being rotatable relative to the upper joint part (2) around a joint axis (5), and brake means (14, 15, 20, 21) having a first force transmission element (14) being coaxially arranged to the joint axis (5), on which a force is acting, which releases the braking action and converts the force by a predetermined transfer ratio to a second force transmission element (15) which is coaxially arranged to the joint axis (5) and exerts the converted force substantially in a parallel direction with respect to the joint axis (5) to a brake disc (20, 21),
**characterized in that**
the first force transmission element (14) and the second force transmission element (15) comprise opposing end faces (14c, 15c), each comprising spindle-shaped increasing and revolving surfaces (14b, 15b).

2. Knee joint with brake according to claim 1, wherein the first and the second force transmission elements (14, 15) form an inclined plane to convert the force.

3. Knee joint with brake according to anyone of claims 1 to 2, comprising an intermediate joint part (4) provided between the upper joint part (2) and the lower joint part (3), wherein the intermediate joint part (4) is either connected with the upper joint part (2) or the lower joint part (3) via the joint axis (5); and is connected with the other one via a swing axis (6).

4. Knee joint with brake according to claim 3, wherein the swing axis (6) is arranged in parallel and on the expanding side with respect to the joint axis (5) and on the side of the lower joint part (3).

5. Knee joint with brake according to anyone of claims 1 to 4, wherein the first and the second force transmission elements (14, 15) are formed such that the first force transmission element (14) is rotated by the force, which releases the brake action, around a rotation axis which is substantially in parallel to the joint axis (5), and that the converted force transfers the second force transmission element (15) in a translation which is substantially in parallel to the joint axis (5).

6. Knee joint with brake according to anyone of claims 1 to 5, wherein the first force transmission element (14) and the second force transmission element (15) form a lifting spindle arranged on the joint axis (5).

7. Knee joint with brake according to anyone of claims 1 to 4, wherein the force transmission takes place by a hydraulic system.

8. Knee joint with brake according to anyone of claims 1 to 4, wherein the force transmission takes place by an angle lever.

9. Knee joint with brake according to anyone of claims 1 to 8, wherein brake discs (20, 21) are provided in the direction of the joint axis (5) on both sides of the second force transmission element (15).

10. Knee joint with brake according to anyone of claims 1 to 9, wherein the presetting of the force, which releases the brake action, is achieved by a biased spring (18).

11. Knee joint with brake according to anyone of claims 1 to 10, wherein piston-cylinder means (7) are provided to damp the walking motion.

12. Knee joint with brake according to anyone of claims 1 to 11, wherein the brake means (14, 15, 20, 21) is controllable by foot load.

13. Knee joint with brake according to anyone of claims 1 to 12, wherein the force acting on the first force transmission element (14) substantially acts vertically with respect to the joint axis.

## Revendications

1. Articulation de genou à frein pour une prothèse de jambe, comportant une partie supérieure (2), une partie inférieure (3) pouvant tourner autour d'un axe d'articulation (5) par rapport à la partie supérieure (2), et un système de frein (14, 15, 20, 21), qui comporte un premier élément de transmission de force (14), qui est disposé coaxialement à l'axe d'articulation (5) et sur lequel agit une force déclenchant l'action de freinage et qui transpose la force moyennant un rapport de transmission prédéfini sur un deuxième élément de transmission de force (15), qui est disposé coaxialement à l'axe d'articulation (5) et qui exerce la force transposée sur un disque de frein (20, 21) dans une direction sensiblement parallèle par référence à l'axe d'articulation (5),
**caractérisée en ce que** le premier élément de transmission de force (14) et le deuxième élément de transmission de force (15) comportent des faces frontales (14c, 15c) orientées l'une vers l'autre, lesquelles comportent des surfaces (14d, 15d) périphériques, montant en forme de spirale.

2. Articulation de genou à frein selon la revendication 1, dans laquelle le premier et le deuxième élément de transmission de force (14, 15) forment un plan incliné pour la transposition de la force.

3. Articulation de genou à frein selon l'une quelconque des revendications 1 à 2, comportant une partie centrale (4), prévue entre la partie supérieure (2) et la partie inférieure (3), ladite partie centrale (4) étant reliée soit avec la partie supérieure (2), soit avec la partie inférieure (3) par l'intermédiaire de l'axe d'articulation (5) et étant reliée avec l'autre partie respective par l'intermédiaire d'un axe de pivotement (6).

4. Articulation de genou à frein selon la revendication 3, dans laquelle l'axe de pivotement (6) est disposé parallèlement par rapport à l'axe d'articulation (5), du côté extension et sur le côté de la partie inférieure (3).

5. Articulation de genou à frein selon l'une quelconque des revendications 1 à 4, dans laquelle le premier et le deuxième élément de transmission de force (14, 15) sont réalisés de telle sorte que, sous l'effet de la force déclenchant l'action de freinage, le premier élément de transmission de force (14) est mis en rotation autour d'un axe de rotation sensiblement parallèle à l'axe d'articulation (5), et la force transposée déplace le deuxième élément de transmission de force (15) selon un mouvement de translation sensiblement parallèle à l'axe d'articulation (5).

6. Articulation de genou à frein selon l'une quelconque des revendications 1 à 5, dans laquelle le premier élément de transmission de force (14) et le deuxième élément de transmission de force (15) forment une vis de levage agencée sur l'axe d'articulation (5).

7. Articulation de genou à frein selon l'une quelconque des revendications 1 à 4, dans laquelle la transmission de force est assurée par l'intermédiaire d'un système hydraulique.

8. Articulation de genou à frein selon l'une quelconque des revendications 1 à 4, dans laquelle la transmission de force est assurée par l'intermédiaire d'un levier coudé.

9. Articulation de genou à frein selon l'une quelconque des revendications 1 à 8, dans laquelle des disques de frein (20, 21) sont prévus dans la direction de l'axe d'articulation (5) de part et d'autre du deuxième élément de transmission de force (15).

10. Articulation de genou à frein selon l'une quelconque des revendications 1 à 9, dans laquelle le préréglage de la force déclenchant l'action de freinage est obtenu par un ressort (18) précontraint.

11. Articulation de genou à frein selon l'une quelconque des revendications 1 à 10, dans laquelle un système à piston et cylindre (7) est prévu pour l'amortissement pendant le mouvement de marche.

12. Articulation de genou à frein selon l'une quelconque des revendications 1 à 11, dans laquelle le système de frein (14, 15, 20, 21) peut être commandé par une sollicitation du pied.

13. Articulation de genou à frein selon l'une quelconque des revendications 1 à 12, dans laquelle la force agissant sur le premier élément de transmission de force (14) agit sensiblement perpendiculairement par référence à l'axe d'articulation.
